# EUROPEAN PATENT APPLICATION

(11) **EP 2 557 176 A1**
(43) Date of publication of application: **13.02.2013**
(21) Application number: 11170011.8
(22) Date of filing: 15.06.2011
(51) Int. Cl.: C12P 13/00, C12P 17/04

(54) **Enzymatic amination**

(71) Applicant: Evonik Degussa GmbH, 45128 Essen (DE)
(72) Inventor: Haas, Thomas, 48161 Münster (DE); Pfeffer, Jan Christoph, 45355 Essen (DE); Faber, Kurt, 8010 Graz (AT); Fuchs, Michael, 8010 Graz (AT)

(57) **Abstract**

A method comprising the steps (a) contacting a hydrocarbon comprising a hydroxyl group with a biological agent having oxygen-dependent and cofactor-dependent carbohydrate oxidase activity in the presence of oxygen and carbohydrate oxidase cofactor, and (b) contacting the hydrocarbon produced in step a) with a biological agent having transaminase activity and a biological agent having cofactor-dependent amino acid dehydrogenase activity in the presence of amino acid dehydrogenase cofactor and the substrate amino acid of the amino acid dehydrogenase.

SEQUENCE LISTING

<110>  Evonik Degussa GmbH

<120>  Enzymatic Amination

<130>  2011E00089 DE

<160>  6

<170>  PatentIn version 3.5

<210>  1
<211>  1935
<212>  DNA
<213>  Artificial Sequence

<220>
<223>  Plasmid for E. coli

<400>  1
catatgatgg cgagcgcgcc gattggcagc gcgattccgc gcaacaactg ggcggtgacc      60

tgcgatagcg cgcagagcgg caacgaatgc aacaaagcga ttgatggcaa caaagatacc     120

ttttggcata ccttttatgg cgcgaacggc gatccgaaac cgccgcatac ctataccatt     180

gatatgaaaa ccacccagaa cgtgaacggc ctgagcgtgc tgccgcgcca ggatggcaac     240

cagaacggct ggattggccg ccatgaagtg tatctgagca gcgatggcac caactggggc     300

agcccggtgg cgagcggcag ctggtttgcg gatagcacca ccaaatatag caactttgaa     360

acccgcccgg cgcgctatgt gcgcctggtg gcgattaccg aagcgaacgg ccagccgtgg     420

accagcattg cggaaattaa cgtgtttcag gcgagcagct ataccgcgcc gcagccgggc     480

ctgggccgct ggggcccgac cattgatctg ccgattgtgc cggcggcggc ggcgattgaa     540

ccgaccagcg gccgcgtgct gatgtggagc agctatcgca acgatgcgtt tgaaggcagc     600

ccgggcggca ttaccctgac cagcagctgg gatccgagca ccggcattgt gagcgatcgc     660

accgtgaccg tgaccaaaca tgatatgttt tgcccgggca ttagcatgga tggcaacggc     720

cagattgtgg tgaccggcgg caacgatgcg aaaaaaacca gcctgtatga tagcagcagc     780

gatagctgga ttccgggccc ggatatgcag gtggcgcgcg gctatcagag cagcgcgacc     840

atgagcgatg gccgcgtgtt taccattggc ggcagctgga gcggcggcgt gtttgaaaaa     900

aacggcgaag tgtatagccc gagcagcaaa acctggacca gcctgccgaa cgcgaaagtg     960

aacccgatgc tgaccgcgga taaacagggc ctgtatcgca gcgataacca tgcgtggctg    1020

tttggctgga aaaaaggcag cgtgtttcag gcgggcccga gcaccgcgat gaactggtat    1080

tataccagcg gcagcggcga tgtgaaaagc gcgggcaaac gccagagcaa ccgcggcgtg    1140

gcgccggatg cgatgtgcgg caacgcggtg atgtatgatg cggtgaaagg caaaattctg    1200

acctttggcg gcagcccgga ttatcaggat agcgatgcga ccaccaacgc gcatattatt    1260

accctgggcg aaccgggcac cagcccgaac accgtgtttg cgagcaacgg cctgtatttt    1320

gcgcgcacct ttcataccag cgtggtgctg ccggatggca gcacctttat taccggcggc    1380

cagcgccgcg gcattccgtt tgaagatagc accccggtgt ttaccccgga aatttatgtg    1440

ccggaacagg atacctttta taaacagaac ccgaacagca ttgtgcgcgc gtatcatagc    1500

attagcctgc tgctgccgga tggccgcgtg tttaacggcg gcggcggcct gtgcggcgat 1560

tgcaccacca accattttga tgcgcagatt tttaccccga actatctgta tgatagcaac 1620

ggcaacctgg cgacccgccc gaaaattacc cgcaccagca cccagagcgt gaaagtgggc 1680

ggccgcatta ccattagcac cgatagcagc attagcaaag cgagcctgat tcgctatggc 1740

accgcgaccc ataccgtgaa caccgatcag cgccgcattc cgctgaccct gaccaacaac 1800

ggcggcaaca gctatagctt tcaggtgccg agcgatagcg gcgtggcgct gccgggctat 1860

tggatgctgt ttgtgatgaa cagcgcgggc gtgccgagcg tggcgagcac cattcgcgtg 1920

acccagtaac tcgag 1935

<210> 2
<211> 1881
<212> DNA
<213> Artificial sequence

<220>
<223> pyranose oxidase from Phanerochaete chrysosporium

<400> 2
catatgatgt tcctagacac aacaccattc agagcagacg aaccatacga cgtattcata 60

gcaggaagcg gaccaatagg agcaacattc gcaaaactat gcgtagacgc aaacctaaga 120

gtatgcatgg tagaaatagg agcagcagac agcttcacaa gcaaaccaat gaaaggagac 180

ccaaacgcac caagaagcgt acaattcgga ccaggacaag taccaatacc aggataccac 240

aaaaaaaacg aaatagaata ccaaaaagac atagacagat tcgtaaacgt aataaaagga 300

gcactaagca catgcagcat accaacaagc aacaaccaca tagcaacact agacccaagc 360

gtagtaagca acagcctaga caaaccattc ataagcctag gaaaaaaccc agcacaaaac 420

ccattcgtaa acctaggagc agaagcagta acaagaggag taggaggaat gagcacacac 480

tggacatgcg caacaccaga attcttcgca ccagcagact tcaacgcacc acacagagaa 540

agaccaaaac taagcacaga cgcagcagaa gacgcaagaa tatggaaaga cctatacgca 600

caagcaaaag aaataatagg aacaagcaca acagaattcg accacagcat aagacacaac 660

ctagtactaa gaaaatacaa cgacatattc caaaaagaaa acgtaataag agaattcagc 720

ccactaccac tagcatgcca cagactaaca gacccagact acgtagaatg gcacgcaaca 780

gacagaatac tagaagaact attcacagac ccagtaaaaa gaggaagatt cacactacta 840

acaaaccaca gatgcacaaa actagtattc aaacactaca gaccaggaga agaaaacgaa 900

gtagactacg cactagtaga agacctacta ccacacatgc aaaacccagg aaacccagca 960

agcgtaaaaa aaatatacgc aagaagctac gtagtagcat gcggagcagt agcaacagca 1020

caagtactag caaacagcca cataccacca gacgacgtag taataccatt cccaggagga 1080

gaaaaaggaa gcggaggagg agaaagagac gcaacaatac caacaccact aatgccaatg 1140

ctaggaaaat acataacaga acaaccaatg acattctgcc aagtagtact agacagcagc 1200

ctaatggaag tagtaagaaa cccaccatgg ccaggactag actggtggaa agaaaaagta 1260

gcaagacacg tagaagcatt cccaaacgac ccaataccaa taccattcag agacccagaa 1320

ccacaagtaa caataaaatt cacagaagaa cacccatggc acgtacaaat acacagagac 1380

gcattcagct acggagcagt agcagaaaac atggacacaa gagtaatagt agactacaga 1440

ttcttcggat acacagaacc acaagaagca aacgaactag tattccaaca acactacaga 1500

gacgcatacg acatgccaca accaacattc aaattcacaa tgagccaaga cgacagagca 1560

agagcaagaa gaatgatgga cgacatgtgc aacatagcac taaaaatagg aggataccta 1620

ccaggaagcg aaccacaatt catgacacca ggactagcac tacacctagc aggaacaaca 1680

agatgcggac tagacacaca aaaaacagta ggaaacacac actgcaaagt acacaacttc 1740

aacaacctat acgtaggagg aaacggagta atagaaacag gattcgcagc aaacccaaca 1800

ctaacaagca tatgctacgc aataagagca agcaacgaca taatagcaaa attcggaaga 1860

cacagaggat aataactcga g 1881

<210> 3
<211> 1845
<212> DNA
<213> Artificial sequence

<220>
<223> Hexose oxidase from Chondrus crispus

<400> 3
catatgatga cattcagcat aaaaacaaga aaaaacacag tagacaaact aaaaaacgaa 60

gaactagacc tactagtaat aggaggagga ataacaggag caggagtagc actacaagca 120

acagcaagcg gactaaaaac aggactagta gaaatgcaag acttcgcaga aggaacaagc 180

agcagaagca caaaactagt acacggagga ataagatacc taaaaacatt cgacgtagga 240

gtagtagcag acacagtaca agaaagagca gtagtacaag gaatagcacc acacatacca 300

agaccattcc caatgctact accaatatac gacgacccaa gcagcacatt cgacatgttc 360

agcgtaaaaa tagcaatgga cctatacgac agactagcag gagtaacagg aacacaatac 420

gcaaactaca caataagcag agaagaagta ctacaaagag aaccaaacct aaaaagcgac 480

aaactactag gagcaggagt atacctagac tacgtaaaca acgacgcaag actagtaata 540

gaaaacataa aagaagcaga cgaactagga ggactaatgg caagccacgt aaaagtagta 600

ggagtactac acgacgacaa cggaagaata aacggagtaa aagcaaaaga cctactaaca 660

gacgaagaat tcgaaataaa agcaaaaatg gtaataaaca caacaggacc atgggtaaca 720

aaaacactag gactagacga caaaaacgga gaaggagaag taataagacc aacaaaagga 780

gtacacctag tagtagacag cagcagacta agagtaccac aaccaacata cttcgacagc 840

ggaataggag acggaagaat gatattcgta gtaccaagag aaaacaaaac atacttcgga 900

acaacagaca cagactacac aggagactac aaacacccaa gagtagaaca agcagacgta 960

gactacctac taaaagcaat aaacaacaga tacccagaag cagaaataac aataaacgac 1020

atagaagcaa gctgggcagg actaagacca ctactaagcg gaaacggaag cagcgactac 1080

aacggaggag gaagcaacac aggaaaagta agcgacgaaa gcttcgaaaa cctaataaaa 1140

acagcagtag catacagcaa agacgaagca agcagaggag aagtagaaaa aagcataagc 1200

agcctaaaaa cagcaagcgc agaaaaaaca ctaagcccaa gccaagtaag cagaggaagc 1260

agcctaaaag taggagacga cggactaata acactaagcg gaggaaaaat aacagactac 1320

agaaaaatgg cagcaggagc aatggaacta ataagaaaac tattcaaaga actattcaac 1380

aaagacgtaa gagaagtaga cagcaaaaaa ctacaagtaa gcggaggaca cttcgaccca 1440

acaaacgtag acgaaacaat gaaattctac caaaaaatag caatggacaa aggaatgcca 1500

gcagcagacg cagcagacct agcaaacaga ttcggaagca acacaagcag agtaataagc 1560

tacgcagacg acgtagaagc agcaccagga ctaacactag cagacacact aagcctacac 1620

tacagcgtaa acgaagaaat gacactaaac ctagtagact tcctactaag aagaacaaac 1680

tacgtactat tccacaaaga cgaactaatg atgaaaaaag acgcattcgt agacgaaatg 1740

gcaagaataa tggaatggaa cgaagaagaa aaagcagcag cagcaaaaca actagacgaa 1800

acaatagcag aaagcagcct agcataccta aaataataac tcgag 1845

<210> 4
<211> 1935
<212> DNA
<213> Artificial sequence

<220>
<223> pASK-IBA35+-AlaDH for E. coli

<400> 4
catatgatgg cgagcgcgcc gattggcagc gcgattccgc gcaacaactg ggcggtgacc 60

tgcgatagcg cgcagagcgg caacgaatgc aacaaagcga ttgatggcaa caaagatacc 120

ttttggcata ccttttatgg cgcgaacggc gatccgaaac cgccgcatac ctataccatt 180

gatatgaaaa ccacccagaa cgtgaacggc ctgagcgtgc tgccgcgcca ggatggcaac 240

cagaacggct ggattggccg ccatgaagtg tatctgagca gcgatggcac caactggggc 300

agcccggtgg cgagcggcag ctggtttgcg gatagcacca ccaaatatag caactttgaa 360

acccgcccgg cgcgctatgt gcgcctggtg gcgattaccg aagcgaacgg ccagccgtgg 420

accagcattg cggaaattaa cgtgtttcag gcgagcagct ataccgcgcc gcagccgggc 480

ctgggccgct ggggcccgac cattgatctg ccgattgtgc cggcggcggc ggcgattgaa 540

ccgaccagcg gccgcgtgct gatgtggagc agctatcgca acgatgcgtt tgaaggcagc 600

ccgggcggca ttaccctgac cagcagctgg gatccgagca ccggcattgt gagcgatcgc 660

accgtgaccg tgaccaaaca tgatatgttt tgcccgggca ttagcatgga tggcaacggc 720

cagattgtgg tgaccggcgg caacgatgcg aaaaaaacca gcctgtatga tagcagcagc 780

gatagctgga ttccgggccc ggatatgcag gtggcgcgcg gctatcagag cagcgcgacc 840

atgagcgatg gccgcgtgtt taccattggc ggcagctgga gcggcggcgt gtttgaaaaa 900

aacggcgaag tgtatagccc gagcagcaaa acctggacca gcctgccgaa cgcgaaagtg 960

aacccgatgc tgaccgcgga taaacagggc ctgtatcgca gcgataacca tgcgtggctg 1020

tttggctgga aaaaaggcag cgtgtttcag gcgggcccga gcaccgcgat gaactggtat 1080

tataccagcg gcagcggcga tgtgaaaagc gcgggcaaac gccagagcaa ccgcggcgtg 1140

gcgccggatg cgatgtgcgg caacgcggtg atgtatgatg cggtgaaagg caaaattctg 1200

acctttggcg gcagcccgga ttatcaggat agcgatgcga ccaccaacgc gcatattatt 1260

accctgggcg aaccgggcac cagcccgaac accgtgtttg cgagcaacgg cctgtatttt 1320

gcgcgcacct ttcataccag cgtggtgctg ccggatggca gcacctttat taccggcggc 1380

cagcgccgcg gcattccgtt tgaagatagc accccggtgt ttaccccgga aatttatgtg 1440

ccggaacagg atacctttta taaacagaac ccgaacagca ttgtgcgcgc gtatcatagc 1500

attagcctgc tgctgccgga tggccgcgtg tttaacggcg gcggcggcct gtgcggcgat 1560

tgcaccacca accattttga tgcgcagatt tttaccccga actatctgta tgatagcaac 1620

ggcaacctgg cgacccgccc gaaaattacc cgcaccagca cccagagcgt gaaagtgggc 1680

ggccgcatta ccattagcac cgatagcagc attagcaaag cgagcctgat tcgctatggc 1740

accgcgaccc ataccgtgaa caccgatcag cgccgcattc cgctgaccct gaccaacaac 1800

ggcggcaaca gctatagctt tcaggtgccg agcgatagcg gcgtggcgct gccgggctat 1860

tggatgctgt ttgtgatgaa cagcgcgggc gtgccgagcg tggcgagcac cattcgcgtg 1920

acccagtaac tcgag 1935

<210> 5
<211> 4534
<212> DNA
<213> Artificial sequence

<220>
<223> pASK-IBA35+-Vflu

<400> 5
ccatcgaatg gccagatgat taattcctaa tttttgttga cactctatca ttgatagagt 60

tattttacca ctccctatca gtgatagaga aaagtgaaat gaatagttcg acaaaaatct 120

agaaataatt ttgtttaact ttaagaagga gatatacaaa tggctagcag aggatcgcat 180

caccatcacc atcacggcgc caacaaaccg cagagctggg aagcgcgtgc ggaaacctat 240

agcctgtatg gctttaccga tatgccgagc ctgcatcagc gtggcaccgt tgttgttacc 300

catggcgaag gcccgtatat cgttgatgtt aacggccgtc gttatctgga tgcgaatagc 360

ggcctgtgga atatggttgc gggctttgat cataaaggcc tgatcgatgc ggcgaaagcg 420

cagtatgaac gttttccggg ctatcatgcg ttttttggcc gtatgagcga tcagaccgtg 480

atgctgagcg aaaaactggt tgaagtgagc ccgtttgata gcggccgtgt gttttatacc 540

aacagcggca gcgaagcgaa tgataccatg gttaaaatgc tgtggtttct gcatgcggcg 600

gaaggtaaac cgcagaaacg taaaattctg acccgctgga acgcgtatca tggcgttacc 660

gcggttagcg cgagcatgac cggcaaaccg tataacagcg tgtttggtct gccgctgccg 720

ggttttgttc atctgacctg cccgcattat tggcgttatg gcgaagaagg cgaaaccgaa 780

gaacagtttg ttgcgcgtct ggcgcgtgaa ctggaagaaa ccattcagcg cgaaggcgcg 840

gataccattg cgggtttttt tgcggaaccg gttatgggtg cgggtggtgt tattccgccg 900

gcgaaaggtt attttcaggc gattctgccg atcctgcgca aatatgatat tccggtgatc 960

agcgatgaag tgatttgcgg ctttggtcgt accggtaata cctggggttg cgtgacctat 1020

gattttaccc cggatgcgat cattagcagc aaaaacctga ccgcgggctt ttttccgatg 1080

ggtgcggtta ttctgggtcc ggaactgagc aaacgtctgg aaaccgcgat tgaagcgatc 1140
gaagaatttc cgcatggttt taccgcgagc ggtcatccgg ttggttgtgc gattgcgctg 1200
aaagcgatcg atgtggttat gaacgaaggc ctggcggaaa atgttcgtcg tctggcgccg 1260
cgttttgaag aacgcctgaa acatattgcg gaacgtccga acattggcga atatcgcggc 1320
attggtttta tgtgggcgct ggaagcggtt aaagataaag cgagcaaaac cccgtttgat 1380
ggcaatctga gcgtgagcga acgtattgcg aatacctgca ccgatctggg tctgatttgt 1440
cgtccgctgg gtcagagcgt tgttctgtgc ccgccgttta ttctgaccga agcgcagatg 1500
gatgaaatgt tcgataaact ggaaaaagcg ctggataaag ttttcgcgga agttgcgtaa 1560
gcttgacctg tgaagtgaaa aatggcgcac attgtgcgac attttttttg tctgccgttt 1620
accgctactg cgtcacggat ctccacgcgc cctgtagcgg cgcattaagc gcggcgggtg 1680
tggtggttac gcgcagcgtg accgctacac ttgccagcgc cctagcgccc gctcctttcg 1740
ctttcttccc ttcctttctc gccacgttcg ccggctttcc ccgtcaagct ctaaatcggg 1800
ggctcccttt agggttccga tttagtgctt tacggcacct cgaccccaaa aaacttgatt 1860
agggtgatgg ttcacgtagt gggccatcgc cctgatagac ggtttttcgc cctttgacgt 1920
tggagtccac gttctttaat agtggactct tgttccaaac tggaacaaca ctcaacccta 1980
tctcggtcta ttcttttgat ttataaggga ttttgccgat ttcggcctat tggttaaaaa 2040
atgagctgat ttaacaaaaa tttaacgcga attttaacaa aatattaacg cttacaattt 2100
caggtggcac ttttcgggga aatgtgcgcg gaacccctat ttgtttattt ttctaaatac 2160
attcaaatat gtatccgctc atgagacaat aaccctgata aatgcttcaa taatattgaa 2220
aaaggaagag tatgagtatt caacatttcc gtgtcgccct tattcccttt tttgcggcat 2280
tttgccttcc tgtttttgct cacccagaaa cgctggtgaa agtaaaagat gctgaagatc 2340
agttgggtgc acgagtgggt tacatcgaac tggatctcaa cagcggtaag atccttgaga 2400
gttttcgccc cgaagaacgt tttccaatga tgagcacttt taaagttctg ctatgtggcg 2460
cggtattatc ccgtattgac gccgggcaag agcaactcgg tcgccgcata cactattctc 2520
agaatgactt ggttgagtac tcaccagtca cagaaaagca tcttacggat ggcatgacag 2580
taagagaatt atgcagtgct gccataacca tgagtgataa cactgcggcc aacttacttc 2640
tgacaacgat cggaggaccg aaggagctaa ccgctttttt gcacaacatg ggggatcatg 2700
taactcgcct tgatcgttgg gaaccggagc tgaatgaagc cataccaaac gacgagcgtg 2760
acaccacgat gcctgtagca atggcaacaa cgttgcgcaa actattaact ggcgaactac 2820
ttactctagc ttcccggcaa caattgatag actggatgga ggcggataaa gttgcaggac 2880
cacttctgcg ctcggccctt ccggctggct ggtttattgc tgataaatct ggagccggtg 2940
agcgtggctc tcgcggtatc attgcagcac tggggccaga tggtaagccc tcccgtatcg 3000
tagttatcta cacgacgggg agtcaggcaa ctatggatga acgaaataga cagatcgctg 3060
agataggtgc ctcactgatt aagcattggt aggaattaat gatgtctcgt ttagataaaa 3120
gtaaagtgat taacagcgca ttagagctgc ttaatgaggt cggaatcgaa ggtttaacaa 3180
cccgtaaact cgcccagaag ctaggtgtag agcagcctac attgtattgg catgtaaaaa 3240

ataagcgggc tttgctcgac gccttagcca ttgagatgtt agataggcac catactcact 3300

tttgcccttt agaaggggaa agctggcaag attttttacg taataacgct aaaagtttta 3360

gatgtgcttt actaagtcat cgcgatggag caaaagtaca tttaggtaca cggcctacag 3420

aaaaacagta tgaaactctc gaaaatcaat tagccttttt atgccaacaa ggtttttcac 3480

tagagaatgc attatatgca ctcagcgcag tggggcattt tactttaggt tgcgtattgg 3540

aagatcaaga gcatcaagtc gctaaagaag aaagggaaac acctactact gatagtatgc 3600

cgccattatt acgacaagct atcgaattat ttgatcacca aggtgcagag ccagccttct 3660

tattcggcct tgaattgatc atatgcggat tagaaaaaca acttaaatgt gaaagtgggt 3720

cttaaaagca gcataacctt tttccgtgat ggtaacttca ctagtttaaa aggatctagg 3780

tgaagatcct ttttgataat ctcatgacca aaatccctta acgtgagttt tcgttccact 3840

gagcgtcaga ccccgtagaa aagatcaaag gatcttcttg agatcctttt tttctgcgcg 3900

taatctgctg cttgcaaaca aaaaaaccac cgctaccagc ggtggtttgt ttgccggatc 3960

aagagctacc aactcttttt ccgaaggtaa ctggcttcag cagagcgcag ataccaaata 4020

ctgtccttct agtgtagccg tagttaggcc accacttcaa gaactctgta gcaccgccta 4080

catacctcgc tctgctaatc ctgttaccag tggctgctgc cagtggcgat aagtcgtgtc 4140

ttaccgggtt ggactcaaga cgatagttac cggataaggc gcagcggtcg ggctgaacgg 4200

ggggttcgtg cacacagccc agcttggagc gaacgaccta caccgaactg agatacctac 4260

agcgtgagct atgagaaagc gccacgcttc ccgaagggag aaaggcggac aggtatccgg 4320

taagcggcag ggtcggaaca ggagagcgca cgagggagct tccaggggga aacgcctggt 4380

atctttatag tcctgtcggg tttcgccacc tctgacttga gcgtcgattt ttgtgatgct 4440

cgtcaggggg gcggagccta tggaaaaacg ccagcaacgc ggccttttta cggttcctgg 4500

ccttttgctg gccttttgct cacatgaccc gaca 4534

<210> 6
<211> 4533
<212> DNA
<213> Artificial sequence

<220>
<223> pASK-IBA5+-pCR6:

<400> 6
ccatcgaatg gccagatgat taattcctaa tttttgttga cactctatca ttgatagagt 60

tattttacca ctccctatca gtgatagaga aaagtgaaat gaatagttcg acaaaaatct 120

agaaataatt ttgtttaact ttaagaagga gatatacaaa tggctagctg gagccacccg 180

cagttcgaaa aaggcgccaa ccaaccgcaa agctgggaag cccgggccga gacctattcg 240

ctctacggtt tcaccgacat gccctcggtc catcagcggg gcacggtcgt cgtgacccat 300

ggcgaggggc cctatatcgt cgatgtccat ggccgccgct atctggatgc caattcgggc 360

ctgtggaaca tggtcgcggg cttcgaccac aagggcctga tcgaggccgc caaggcgcaa 420

tacgaccgct ttcccggcta tcacgccttt ttcggccgca tgtccgacca gaccgtgatg 480

ctgtcggaaa agctggtcga ggtctcgcca ttcgacaacg gccgggtctt ctataccaat 540
tccggctccg aggcgaacga caccatggtc aagatgctgt ggttcctgca tgccgccgag 600
ggcaagccgc aaaagcgcaa gatcctgacg cgctggaacg cctatcacgg cgtgaccgcg 660
gtttcggcct cgatgaccgg caagccctac aactcggtct tcggcctgcc gctgcccggc 720
ttcatccacc tgacctgccc gcattactgg cgctatggcg aggaaggcga gaccgaggcg 780
caattcgtcg cccgcctggc acgcgagctt gaggatacca tcacccgcga gggcgccgac 840
accatcgccg gcttcttcgc cgagccggtg atgggcgcgg ggggggtgat cccgccggcg 900
aagggttatt tccaggccat cctgccgatc ttgcgcaagt atgacatccc gatgatctcg 960
gacgaggtga tctgcggctt cgggcgcacc ggcaacacct ggggctgcct gacctacgac 1020
ttcatgcccg atgcgatcat ctcgtccaag aacctgactg cgggcttctt cccgatgggc 1080
gccgtcatcc tcgggcccga cctcgccaag cgggtcgagg ccgcggtcga ggcgatcgag 1140
gagttcccgc acggcttcac cgcctcgggc catccggtcg gctgcgccat cgcgctgaag 1200
gccatcgacg tggtgatgaa cgaggggctg gccgagaatg tccgccgcct cgcaccccgc 1260
ttcgaggcgg ggctgaagcg catcgccgac cgcccgaaca tcggcgaata ccgcggcatc 1320
ggcttcatgt gggcgctgga ggcggtcaag gacaagccga ccaagacccc cttcgacgcc 1380
aatctttcgg tcagcgagcg catcgccaat acctgcaccg atctggggct gatctgccgg 1440
ccgctgggcc agtccatcgt gctgtgcccg cccttcatcc tgaccgaggc gcagatggac 1500
gagatgttcg aaaagctgga aaaggcgctc gacaaggtct ttgccgaggt ggcctaaaag 1560
cttgacctgt gaagtgaaaa atggcgcaca ttgtgcgaca ttttttttgt ctgccgttta 1620
ccgctactgc gtcacggatc tccacgcgcc ctgtagcggc gcattaagcg cggcgggtgt 1680
ggtggttacg cgcagcgtga ccgctacact tgccagcgcc ctagcgcccg ctcctttcgc 1740
tttcttccct tcctttctcg ccacgttcgc cggctttccc cgtcaagctc taaatcgggg 1800
gctcccttta gggttccgat ttagtgcttt acggcacctc gaccccaaaa aacttgatta 1860
gggtgatggt tcacgtagtg ggccatcgcc ctgatagacg gtttttcgcc ctttgacgtt 1920
ggagtccacg ttctttaata gtggactctt gttccaaact ggaacaacac tcaaccctat 1980
ctcggtctat tcttttgatt tataagggat tttgccgatt tcggcctatt ggttaaaaaa 2040
tgagctgatt taacaaaaat ttaacgcgaa ttttaacaaa atattaacgc ttacaatttc 2100
aggtggcact tttcggggaa atgtgcgcgg aacccctatt tgtttatttt tctaaataca 2160
ttcaaatatg tatccgctca tgagacaata accctgataa atgcttcaat aatattgaaa 2220
aaggaagagt atgagtattc aacatttccg tgtcgccctt attccctttt ttgcggcatt 2280
ttgccttcct gtttttgctc acccagaaac gctggtgaaa gtaaaagatg ctgaagatca 2340
gttgggtgca cgagtgggtt acatcgaact ggatctcaac agcggtaaga tccttgagag 2400
ttttcgcccc gaagaacgtt ttccaatgat gagcactttt aaagttctgc tatgtggcgc 2460
ggtattatcc cgtattgacg ccgggcaaga gcaactcggt cgccgcatac actattctca 2520
gaatgacttg gttgagtact caccagtcac agaaaagcat cttacggatg gcatgacagt 2580
aagagaatta tgcagtgctg ccataaccat gagtgataac actgcggcca acttacttct 2640

gacaacgatc ggaggaccga aggagctaac cgcttttttg cacaacatgg gggatcatgt 2700
aactcgcctt gatcgttggg aaccggagct gaatgaagcc ataccaaacg acgagcgtga 2760
caccacgatg cctgtagcaa tggcaacaac gttgcgcaaa ctattaactg gcgaactact 2820
tactctagct tcccggcaac aattgataga ctggatggag gcggataaag ttgcaggacc 2880
acttctgcgc tcggcccttc cggctggctg gtttattgct gataaatctg gagccggtga 2940
gcgtggctct cgcggtatca ttgcagcact ggggccagat ggtaagccct cccgtatcgt 3000
agttatctac acgacgggga gtcaggcaac tatggatgaa cgaaatagac agatcgctga 3060
gataggtgcc tcactgatta agcattggta ggaattaatg atgtctcgtt tagataaaag 3120
taaagtgatt aacagcgcat tagagctgct taatgaggtc ggaatcgaag gtttaacaac 3180
ccgtaaactc gcccagaagc taggtgtaga gcagcctaca ttgtattggc atgtaaaaaa 3240
taagcgggct ttgctcgacg ccttagccat tgagatgtta gataggcacc atactcactt 3300
ttgcccttta gaaggggaaa gctggcaaga ttttttacgt aataacgcta aaagttttag 3360
atgtgcttta ctaagtcatc gcgatggagc aaaagtacat ttaggtacac ggcctacaga 3420
aaaacagtat gaaactctcg aaaatcaatt agccttttta tgccaacaag gtttttcact 3480
agagaatgca ttatatgcac tcagcgcagt ggggcatttt actttaggtt gcgtattgga 3540
agatcaagag catcaagtcg ctaaagaaga aagggaaaca cctactactg atagtatgcc 3600
gccattatta cgacaagcta tcgaattatt tgatcaccaa ggtgcagagc cagccttctt 3660
attcggcctt gaattgatca tatgcggatt agaaaaacaa cttaaatgtg aaagtgggtc 3720
ttaaaagcag cataaccttt ttccgtgatg gtaacttcac tagtttaaaa ggatctaggt 3780
gaagatcctt tttgataatc tcatgaccaa aatcccttaa cgtgagtttt cgttccactg 3840
agcgtcagac cccgtagaaa agatcaaagg atcttcttga gatccttttt ttctgcgcgt 3900
aatctgctgc ttgcaaacaa aaaaaccacc gctaccagcg gtggtttgtt tgccggatca 3960
agagctacca actctttttc cgaaggtaac tggcttcagc agagcgcaga taccaaatac 4020
tgtccttcta gtgtagccgt agttaggcca ccacttcaag aactctgtag caccgcctac 4080
atacctcgct ctgctaatcc tgttaccagt ggctgctgcc agtggcgata agtcgtgtct 4140
taccgggttg gactcaagac gatagttacc ggataaggcg cagcggtcgg gctgaacggg 4200
gggttcgtgc acacagccca gcttggagcg aacgacctac accgaactga gatacctaca 4260
gcgtgagcta tgagaaagcg ccacgcttcc cgaagggaga aaggcggaca ggtatccggt 4320
aagcggcagg gtcggaacag gagagcgcac gagggagctt ccagggggaa acgcctggta 4380
tctttatagt cctgtcgggt ttcgccacct ctgacttgag cgtcgatttt tgtgatgctc 4440
gtcagggggg cggagcctat ggaaaaacgc cagcaacgcg gcctttttac ggttcctggc 4500
cttttgctgg ccttttgctc acatgacccg aca 4533

## Description

The present invention relates to a method comprising the steps a) contacting a hydrocarbon comprising a hydroxy group with a biological agent having oxygen-dependent and cofactor-dependent carbohydrate oxidase activity in the presence of oxygen and carbohydrate oxidase cofactor, and b) contacting the hydrocarbon produced in step a) with a biological agent having transaminase activity and a biological agent having cofactor-dependent amino acid dehydrogenase activity in the presence of amino acid dehydrogenase cofactor and the substrate amino acid of the amino acid dehydrogenase, an aqueous mixture comprising a biological agent having oxygen-dependent and cofactor-dependent carbohydrate oxidase activity, a biological agent having transaminase activity, a biological agent having amino acid dehydrogenase activity, oxygen, the substrate amino acid of the amino acid dehydrogenase, carbohydrate oxidase cofactor and amino acid dehydrogenase cofactor and preferably a biological agent having formate dehydrogenase activity for transaminating a hydrocarbon comprising a hydroxy group, and a use of such an aqueous reaction mixture.

The era of non-renewable fossil fuels is coming to an end. While chemists have been able to rely on a virtually unlimited supply of coal, petroleum and natural gas, the availability of such resources, formed by bacteria, plancton, plant and animal matter buried in ocean sediments over millions of years, is expected to be limiting in the very near future. Moreover, burning fossil fuels has been linked with the increase in atmospheric concentrations of CO₂ and associated climate changes, most notably global warming. Therefore, next generation processes for the production of chemicals conventionally derived from fossil resources will have to start with renewable resources, *i. e*. materials that are easily and, in terms of geological time scales, rapidly replenishable.

Industrial biotechnology, *i. e*. the application of biocatalysts such as enzymes or organisms as industrial catalysts, offers alternatives to many conventional processes using fossil resources as input. Not only are biocatalysts able to convert compounds made from renewable materials, often agricultural or process wastes that would otherwise have to be disposed of, but they do not require the use of toxic compounds and, last but not least, reduce greenhouse gas emissions compared to conventional approaches.

Amines, organic derivates of ammonia, represent of a class of industrially sought-after compounds, including bulk chemicals such as aniline, often prepared by conversion of compounds made from fossil carbon sources such as halogenated alkanes or alkenes. The reactions used for the synthesis of amines depend on the use of extreme temperatures, toxic compounds such as HCN and expensive of environmentally hazardous catalysts. Examples of industrially relevant amines include aniline, a precursor to various industrial chemicals, lysine, an essential amino acid, and di-(aminomethyl)-furan (DAMF), a monomer used for the production of polymers.

The prior art teaches a chemical route towards the synthesis of DAMF and chemically related amines, involving the stepwise oxidation of HMF to DFF, followed by transformation to the corresponding dioxime and subsequent nickel-catalyzed reduction of the latter (El Hajj *et al.,* 1987). A biotechnological route based on renewable materials such as sugars would be desirable.

Therefore, the problem underlying the present invention is to provide a system that may be used to produce amines starting with compounds comprising a hydroxyl group, preferably attached to an aromatic or aliphatic carbon ring, which compounds may be derived from renewable starting materials rather than compounds obtained by way of cracking fossil resources. Another problem underlying the present invention is to provide an entirely enzymatic route towards the production of organic amines, preferably one with a broad substrate specificity and/or improved yield and/or product specificity compared to reactions described in the prior art and one that does not require the addition of hazardous reagents and/or harsh reaction conditions.

Another problem involves the provision of a suite of reactions for the production of amines that has, *in toto,* a redox balance of 0.

Another problem underlying the present invention is to provide a system that may be used to produce amines starting with compounds comprising a hydroxyl group, preferably attached to an aromatic or aliphatic carbon ring, without the need to subject intermediates to purification and/or extraction procedures or have a change of solvent.

The problem underlying the present invention is solved by the subject-matter of the attached independent and dependent claims.

In a first aspect, the problem underlying the present invention is solved by a method comprising the steps
(a) contacting a hydrocarbon comprising a hydroxyl group with
   a biological agent having oxygen-dependent and cofactor-dependent carbohydrate oxidase activity
   in the presence of oxygen and carbohydrate oxidase cofactor, and
(b) contacting the hydrocarbon produced in step a) with
   a biological agent having transaminase activity and
   a biological agent having cofactor-dependent amino acid dehydrogenase activity
   in the presence of amino acid dehydrogenase cofactor and the substrate amino acid of the amino acid dehydrogenase.

In an embodiment of the first aspect, the hydrocarbon comprising a hydroxyl group comprises a 5 or 6 membered ring carrying at least one substituent selected from the group comprising -(CH₂)ₓ-OH, wherein x is 0 to 4.

In an embodiment of the first aspect, the hydrocarbon comprising a hydroxyl group is selected from the group of compounds represented by formulae (I) or (II) 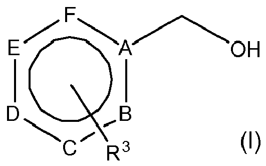 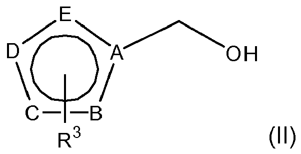 wherein up to two out of A, B, C, D, E and F are atoms each and independently selected from the group comprising N, S and O and the others are C,
wherein R³ is selected from the group comprising H, substituted or unsubstituted alkyl, alkenyl, alkynyl, -(CH₂)ₓ-CR⁴O, -(CH₂)ₓ-NH₂, -(CH₂)ₓ-NO₂, -(CH₂)ₓ-C-O-R⁴, -(CH₂)ₓ-CH₂SH, -(CH₂)ₓ-COOR⁴,
wherein x is 0 to 20, preferably 0 to 4, more preferably 1 to 2,
and wherein R⁴ is selected from the group comprising H and substituted or unsubstituted alkyl, cycloalkyl, aryl and heteroaryl.

In an embodiment of the first aspect, up to two out of A, B, C, D, E and F are atoms each and independently selected from the group comprising N and O and the others are C,
wherein R³ is selected from the group comprising -(CH₂)ₓ-CR⁴O, -(CH₂)ₓ-NH₂, -(CH₂)ₓ-O-R⁴ and -(CH₂)ₓ-C-COOR⁴_{,}
wherein x is 0 to 4, more preferably 1 to 2,
and wherein R⁴ is selected from the group comprising H and alkyl comprising 1 to 4 carbon atoms.

In an embodiment of the first aspect, A, B, C, D, E and F are all C atoms.

In an embodiment of the first aspect, the hydrocarbon comprising a hydroxyl group is a cycloalkanol, preferably cyclohexanol.

In an embodiment of the first aspect, the oxygen-dependent and cofactor-dependent carbohydrate oxidase activity is an activity provided by an enzyme selected from the group comprising the M1 variant of galactose oxidase from *Fusarium* NRRL 2903, pyranose oxidase from *Phanerochaete chrysosporium,* hexose oxidase from *Chondrus crispus* and homologues thereof.

In an embodiment of the first aspect, the amino acid dehydrogenase is alanine dehydrogenase.

In an embodiment of the first aspect, the transaminase is selected from the group comprising the ω-transaminases from *Vibrio fluvialis* and *Paracoccus denitrificans* and homologues thereof.

In an embodiment of the first aspect, step b) is carried out in the presence of a biological agent having formate dehydrogenase activity.

In an embodiment of the first aspect, the redox factor produced by the biological agent having amino acid dehydrogenase activity is consumed by the biological agent having formate dehydrogenase activity.

In an embodiment of the first aspect, step a) is carried out in the presence of an H₂O₂-degrading activity.

In an embodiment of the first aspect, the H₂O₂-degrading activity is provided by a biological agent selected from the group comprising catalase and horse radish peroxidase/ABTS and homologues thereof.

In an embodiment of the first aspect, steps a) and b) are carried out simultaneously in the same reaction mixture.

In an embodiment of the first aspect, at least one component essential for the transaminase activity, preferably the biological agent having transaminase activity, is added to the reaction mixture following addition of the components essential for oxygen-dependent and cofactor-dependent carbohydrate oxidase activity.

In an embodiment of the first aspect, the level of oxygen pressure is 2 to 7 bar, preferably 3 to 5 bar.

In an embodiment of the first aspect, one or more, preferably all, of the biological agents selected from the group comprising the biological agent having oxygen-dependent and cofactor-dependent carbohydrate oxidase activity, the biological agent having transaminase activity, the biological agent having formate dehydrogenase activity and the biological agent having cofactor-dependent alanine dehydrogenase activity and the biological agent having formate dehydrogenase activity are associated with a viable cell.

In a second aspect, the problem underlying the present invention is solved by a use of a mixture comprising a biological agent having oxygen-dependent and cofactor-dependent carbohydrate oxidase activity, a biological agent having transaminase activity, a biological agent having amino acid dehydrogenase activity, oxygen, the substrate amino acid of the amino acid dehydrogenase, carbohydrate oxidase cofactor and amino acid dehydrogenase cofactor and preferably a biological agent having formate dehydrogenase activity for transaminating a hydrocarbon comprising a hydroxyl group, wherein one or more than one, preferably all biological agents are or comprise a heterologous polypeptide having the activity of interest.

In a third aspect, the problem underlying the present invention is solved by an aqueous reaction mixture comprising a hydrocarbon comprising a hydroxyl group, a biological agent having oxygen-dependent and cofactor-dependent carbohydrate oxidase activity, oxygen, carbohydrate oxidase cofactor, a biological agent having transaminase activity, a biological agent having amino acid dehydrogenase, amino acid dehydrogenase cofactor, the substrate amino acid of the amino acid dehydrogenase and preferably a biological agent having formate dehydrogenase activity, wherein one or more than one, preferably all biological agents are or comprise a heterologous polypeptide having the activity of interest.

In a preferred embodiment of the third aspect, each of the biological agents specified is a whole cell biocatalyst comprising one or more heterologous active polypeptides having the activities of interest, preferably one whole cell biocatalyst comprising more than one heterologous active polypeptides has all the activities of interest.

Further embodiments of the second and third aspect of the present invention comprise the embodiments of the first aspect of the present invention.

The present invention is based on the surprising finding that a sequence of reactions exists that may be used to catalyse the oxidation and subsequent transamination of a hydrocarbon comprising a hydroxyl group, wherein said hydroxyl group is converted to an amine group, based entirely on biotechnological catalysts. Moreover, the present inventors have surprisingly found that all of these reactions are fully compatible with each other and may be carried out simultaneously in the same reaction vessel, using a uniform buffer system. Moreover, the present inventors have surprisingly found a suitable sequence of reactions that may be carried out without an *in toto* addition or removal of electrons.

The inventive process may be used to convert a broad range of compounds. In a preferred embodiment, the term "hydrocarbon comprising a hydroxyl group", as used herein, refers to any organic compound comprising two carbon atoms linked *via* a C-C bond and comprising at least one hydroxyl group, the latter preferably attached to a carbon atom. In a preferred embodiment, the carbon chain of such a hydrocarbon is selected from the group comprising cyclic and linear alkanes, alkenes, alkynes, alkyl or alkenyl aryls and alkyl or alkenyl heteroaryls.

In a preferred embodiment, the hydrocarbon comprising a hydroxyl group is selected from the group of compounds represented by formula (III): 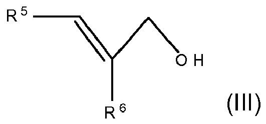 wherein R⁵ and R⁶ are each and independently any chemical group and preferably part of an aromatic ring.

In a preferred embodiment, the hydrocarbon comprising a hydroxyl group is selected from the group of compounds represented by formula (IV): 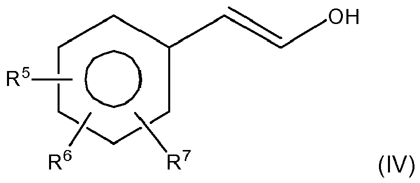 wherein each of R⁵, R⁶ and R⁷ is each and independently selected from the group comprising H, substituted or unsubstituted alkyl, alkenyl, alkynyl, -(CH₂)ₓ-CR⁴O, -(CH₂)ₓ-NH₂, -(CH₂)ₓNO₂, -(CH₂)ₓ-O R⁴, -(CH₂)ₓ-CH₂SH, -(CH₂)ₓ-COOR⁴,
wherein x is 0 to 20, preferably 0 to 4,
and wherein R⁴ is selected from the group comprising H and substituted or unsubstituted alkyl, cycloalkyl, aryl and heteroaryl,
and wherein up to one or two carbon atomes of the aromatic ring may each and independently be replaced by an atom selected from the group comprising N, O and S.

In a most preferred embodiment, the hydrocarbon comprising a hydroxyl group is HMF.

In a preferred embodiment, the term "biological agent", as used herein, refers to any organism or molecule of biological origin that has the activity specified. In a more preferred embodiment, the biological agent is a more or less purified or isolated active biological macromolecule, most preferably a peptide or polypeptide. In another more preferred embodiment, the biological agent is a whole cell biocatalyst comprising or being associated with such a biological macromolecule. In a preferred embodiment, carbohydrate oxidase activity is provided by a carbohydrate oxidase or a homologue thereof. In another preferred embodiment, transaminase activity is provided by a transaminase or homologues thereof. In another preferred embodiment, catalase activity is provided by a catalase or a homologue thereof. In another preferred embodiment, amino acid, preferably alanine, dehydrogenase activity is provided by an amino acid, preferably alanine, dehydrogenase or a homologue thereof. In another preferred embodiment, formate dehydrogenase activity is provided by a formate dehydrogenase or a homologue thereof. The biological agent may be immobilised.

In a preferred embodiment, the term "presence of a cofactor", as used herein, means that a cofactor required for activity or enhanced activity of an activity and/or enzyme is present, either free in solution and/or tightly associated with said activity and/or enzyme.

It is clear that reaction conditions compatible with the activities involved have to be chosen, more specifically pH, buffer, buffer concentration and the like. In a preferred embodiment, the concentration of buffer, preferably potsassium phosphate, is more than 25 mM, preferably 25 to 250 mM, more preferably 40 to 220 mM, most preferably 90 to 160 mM, and the pH is 5 to 9, preferably 6,5 to 8,5.

In a preferred embodiment, the term "activity", as used herein, refers to the ability to catalyse a chemical or biological reaction. In other words, a chemical or biological reaction reaches a state of equilibrium more rapidly if a suitable activity is present. In a preferred embodiment, the activity is displayed by a catalytically active polypeptide or cell comprising such a polypeptide.

In a most preferred embodiment, the biological agent is a biological cell. The person skilled in the art is familiar with a wide range of biological cells that may be used to display a biological activity of interest. For example, the cell may be a prokaryotic cell, preferably a bacterial cell such as an *Escherichia, Corynebaterium* or *Pseudomonas* cell, most preferably an *E*. *coli* cell. The biological cell may also be a eukaryotic cell, preferably a unicellular eukaryotic cell, most preferred a fungal biological cell, for example *Candida tropicalis, Saccharomyces cerevisiae* or *Pichia pastoris.* The person skilled in the art is also familiar with a wide range of techniques that may be used to genetically alter cells such that they express a biological or other activity of interest.

Step a) of the present invention involves the use of a biological agent having carbohydrate oxidase activity. In a preferred embodiment, the term "carbohydrate oxidase activity", as used herein, refers to an enzymatic activity that is able to oxidise a carbohydrate, preferably a carbohydrate capable of forming, at least transiently, a five- or six-membered ring structure comprising at least one hydroxyl group, wherein at least one hydroxyl group is oxidised, preferably such that it is replaced by a carbonyl group. In a preferred embodiment, the term "hydrocarbon produced in step a)", as used herein, means that the hydrocarbon subjected to contact with a biological agent having carbohydrate oxidase activity under the conditions specified with respect to step a) have the opportunity to first interact with the agent having carbohydrate oxidase activity, subsequently the hydrocarbon modified by way of interaction with the carbohydrate oxidase activity may proceed to react with the reagents specified with respect to step b). This does not necessitate that the reagents and activities specified with respect to step b) be absent as step a) occurs.

The person skilled in the art knows how to prepare biological agents having adequate activities, in particular by introducing suitable polypeptides into cellular hosts by means of genetic engineering using standard molecular biology techniques. Moreover, the person skilled in the art knows how to employ such cells as whole-cell catalysts or use them as a starting material to prepare more or less isolated polypeptides having the activity of interest. Finally, the person skilled in the art is familiar with ways to preserve such activities or use it to catalyse chemical reactions, for example by addition essential cofactors, buffer reagents and adjusting pH and temperature conditions in a suitable manner. Finding such conditions is a matter of routine experimentation.

In a preferred embodiment, the carbohydrate oxidase activity is an oxygen-dependent carbohydrate oxidase activity, *i. e*. it depends on the presence of molecular oxygen as an oxidant. The person skilled in the art is aware of methods that may be used to supply oxygen in an aqueous solution, for example by bubbling gaseous oxygen through the aqueous solution or by adding to the aqueous solution oxygen-releasing agents.

The carbohydrate oxidase activity is, aside from oxygen, also dependent on a cofactor. In a preferred embodiment of the present invention, the term "cofactor", as used herein, refers to a redox factor that is either tightly associated with the molecule having the activity of interest or transiently interacting with such a molecule, preferably as a substrate. In a preferred embodiment, the carbohydrate oxidase uses its cofactor as an oxidant, *i. e*. to sink electrons released upon oxidation of the substrate's hydroxyl group. In another preferred embodiment of the present invention, the carbohydrate oxidase cofactor is a flavine cofactor, more preferably FAD⁺. In another preferred embodiment, the cofactor is selected from the group comprising FAD⁺, NAD⁺ and NADP⁺.

Step b) of the present invention involves the use of a biological agent having transaminase activity. In a preferred embodiment, the term "transaminase activity", as used herein, is an activity capable of catalysing the conversion of a carbonyl function to an amine function. In a more preferred embodiment, the term "transamination activity", as used herein, refers to an activity that is able to catalyse the conversion of a carbohydrate having a hydroxyl group to a carbohydrate having an amine group, with the amine function replacing the hydroxyl group.

Step b) also requires participation of an amino acid dehydrogenase activity. In a preferred embodiment, the term "amino acid dehydrogenase activity", as used herein, refers to an activity that is able to catalyse the conversion of an acid comprising a amine function, *i. e*. an amino acid, preferably alanine, to an acid, wherein the amino function is replaced by carbonyl function. In a most preferred embodiment, the amino acid dehydrogenase is an alanine dehydrogenase catalysing the conversion of alanine to pyruvate. In a most preferred embodiment, the amino acid dehydrogenase is dependent on a cofactor. In a preferred embodiment, the amino acid dehydrogenase cofactor is NAD⁺.

Finally, step b) also involves a formate dehydrogenase activity. In a preferred embodiment, the term "formate dehydrogenase activity", as used herein, refers to an activity capable of converting formate to CO₂, wherein a formate dehydrogenase cofactor is reduced. In a preferred embodiment, the formate dehydrogenase cofactor is NAD⁺.

In a preferred embodiment, the term "-CR⁴O", as used herein, refers to an aldehyd substituent in case R⁴ is hydrogen or to a keto substituent if R⁴ is an organic substituent, wherein C is covalently bound to two carbon atoms.

It is a particular strength of the present invention that the sequence of reactions devised by the present inventors has a net redox balance of 0, *i*.*e*. it does *in toto* not require the addition or removal of electrons and/or oxidised or reduced redox cofactors. This is accomplished by employing an amino acid dehydrogenase, preferably an alanine dehydrogenase, that releases, in an oxidised form, the cofactor used by the formate dehydrogenase to oxidase formate.

The present inventors have found that it is advantageous to carry out step a) in the presence of H₂O₂-degrading activity. In a preferred embodiment, the term "H₂O₂-degrading activity", as used herein, refers to any activity that reduces the amount of H₂O₂ present in a given aqueous solution. The person skilled in the art is familiar with many catalysts, of inorganic, organic or even biological origin, that may be used for providing H₂O₂-degrading activity.

Moreover, the present inventors have surprisingly found that the entire suite of reactions presented, *i. e*. no less than five independent chemical reactions, are entirely compatible with respect to each other, enabling the person skilled in the art to carry out all the reactions simultaneously in the same reaction vessel without the need to add or mix components in a particular order or to purify an intermediate.

In a preferred embodiment, homologues of any amino acid or nucleic acid sequences referred to in this application explicitly, for example by name or accession number or indeed the term "homologue of", or implicitly, for example by functional characterisation, are within scope of the present invention. In a preferred embodiment, the term "homologue", as used herein, comprises amino acid or nucleic acid sequences, respectively, that are 60, 70, 75, 80, 85, 90, 92, 94, 95, 96, 97, 98 or 99 % identical to the reference amino acid or nucleic acid sequence. In a preferred embodiment, the term "homologue", with regard to amino acid sequence, comprises, preferably in addition to the above sequence identity, amino acid sequences that comprise one or more conservative amino acid changes with respect to the reference sequence. In a preferred embodiment, the term "homologue" of an amino acid sequence or nucleic acid sequence comprises, preferably in addition to the above sequence identity, active portions and/or fragments of the amino acid sequence or nucleic acid sequence, respectively. In a preferred embodiment, the term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity, *e. g*. as a carbohydrate oxidase, ω-transaminase, catalase, formate dehydrogenase or amino acid dehydrogenase. In a preferred embodiment, the term "retains at least some of its essential biological activity", as used herein, means that the amino acid sequence in question has a biological activity exceeding and distinct from the background activity and the kinetic parameters characterising said activity, more specifically k_{cat} and K_{M}, are preferably within 3, more preferably 2, most preferably one order of magnitude of the values displayed by the reference molecule with respect to a specific substrate. In a preferred embodiment, the term "homologue" of a nucleic acid comprises nucleic acids the complementary strand of which hybridises, preferably under stringent conditions, to the reference nucleic acid. Stringency of hybridisation reactions is readily determinable by one of ordinary skilled in the art, and in generally is an empirical calculation dependent on probe length, washing temperature and salt concentration. In general longer probes require higher temperatures for proper annealing, while shorter probes need lower temperatures. Hybridisation generally depends on the ability of denatured DNA to reanneal to complementary strands are present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see Ausubel *et al.* (1995). In a preferred embodiment, the term "homologue" of a nucleic acid sequence, as used herein, refers to any nucleic acid sequence that encodes the same amino acid sequence as the reference nucleic acid sequence, in line with the degeneracy of the genetic code.

In a preferred embodiment of the present invention, the term "associated with a viable cell", as used herein, means that the biological agent characterised as such is a polypeptide located in the inside of the cell or attached to the surface or to the membrane of a cell. In a more preferred embodiment, the polypeptide is fused to a membrane protein.

In a most preferred embodiment, the hydrocarbon comprising a hydroxyl group is HMF, and the carbohydrate oxidase is preferably the M1 variant of galactose oxidase from *Fusarium* NRRL 2903 (Escalettes & Turner, 2008) or the pyranose oxidase from *Phanerochaete chrysosporiumor* the hexose oxidase from *Chondrus crispus* or a homologue of any of them, and the transaminase is *Vibrio flurialis* or *Paracoccus denitrificans* transaminase or a homologue thereof.

The present invention is further illustrated by the following figures and non-limiting examples from which further features, embodiments, aspects and advantages of the present invention may be taken.

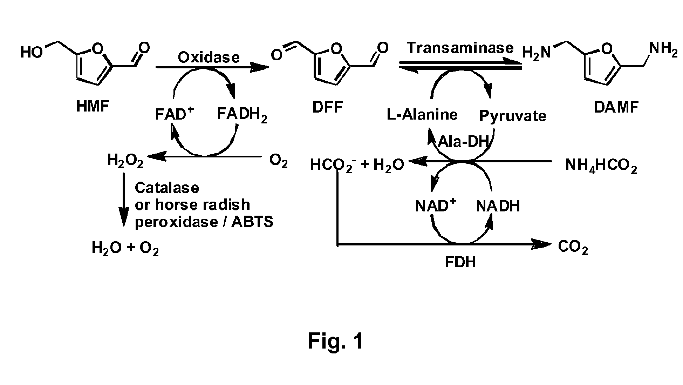

**Fig. 1** depicts a scheme illustrating the inventive suite of reactions.
**Fig. 2** depicts the range of components subjected to oxidation, as described in Example 1, and subsequent transamination, as described in Example 5, in addition to HMF.
**Fig. 3** shows the results of pilot experiments aiming to optimise pH and buffer concentration with respect to the oxidation reaction catalysed by the M1 variant of galactose oxidase from *Fusarium* NRRL2903.
**Fig. 4** shows the results of pilot experiments aiming to optimise the oxygen pressure with respect to the oxidation reaction catalysed by the M1 variant of galactose oxidase from *Fusarium* NRRL2903.

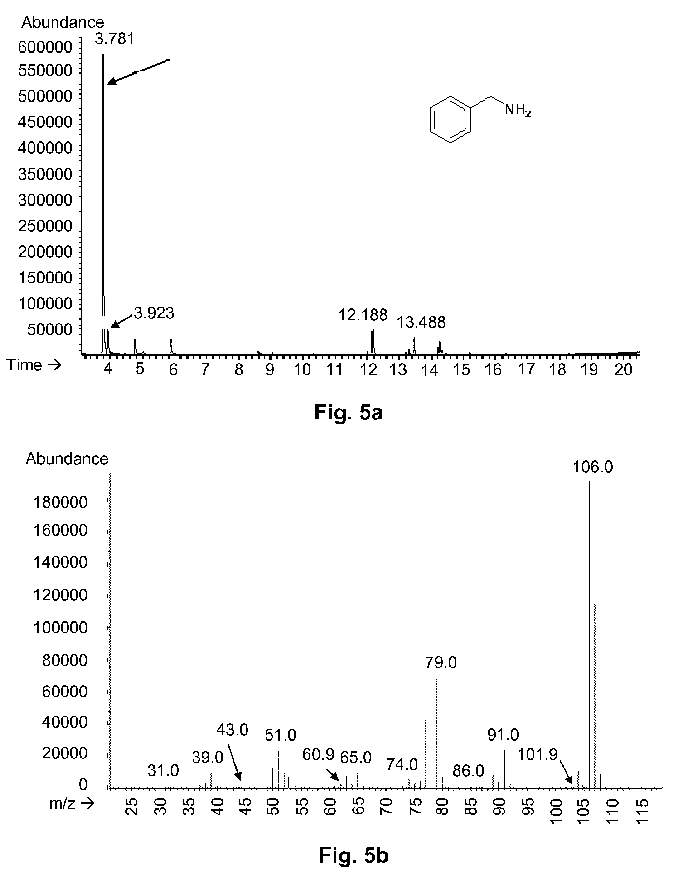

**Figs. 5a** and **5b** show mass spectrometry results demonstrating formation of benzylamine following oxidation and amination of benzylalcohol.

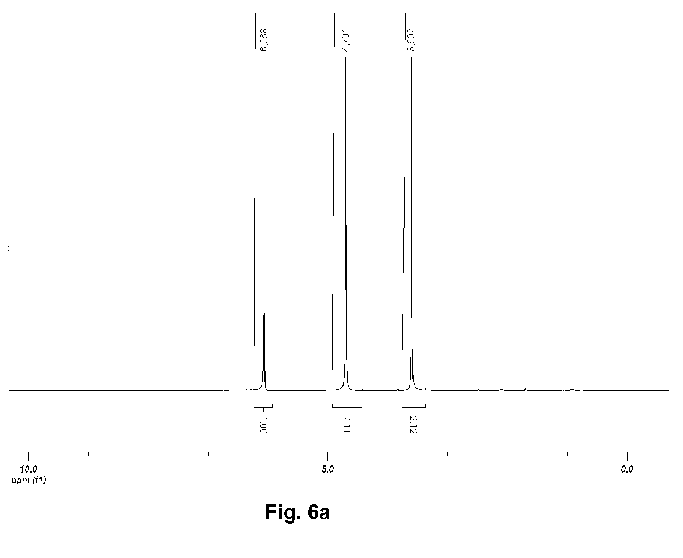

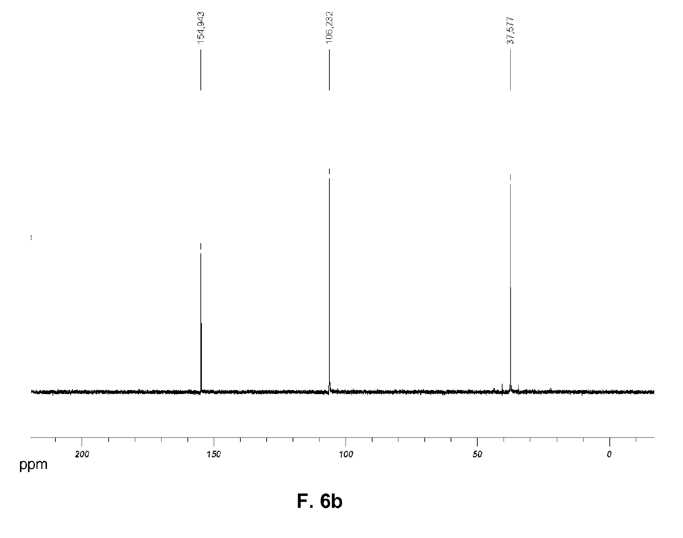

**Fig. 6** shows NMR data demonstrating formation of DAMF following oxidation and amination of
HMF.

### Example 1: Oxidation of various cyclic compounds comprising a hydroxyl function by carbohydrate oxidases

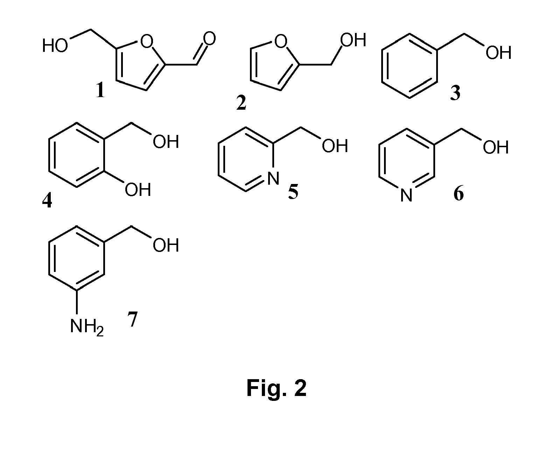

A range of cyclic compounds comprising a hydroxyl group (**Fig. 2**) in addition to HMF was subjected to carbohydrate oxidase oxidation as follows. A whole cell preparation of the corresponding oxidase (20 mg, lyophilised dry weight) was resuspended in the corresponding phosphate buffer [1 mL; standard conditions: galactose oxidase: 100 mM sodium phosphate, pH = 7, CuSO₄.5H₂O (3 mg/mL); pyranose and hexose oxidase: 100 mM sodium phosphate, pH = 8, 1 mM FAD] at 30°C and 120 rpm for 20 min (horizontal position). Afterwards, horseradish peroxidase (Sigma, # P8125) (30 µL, 10 mg/mL), 2,2'-azino-bis(3-ethylbenzthiazoline-6-sulfonic acid) (ABTS) (30 µL, 10 mg/mL) and the respective hydrocarbon, for example HMF (5 mg, 40 mmol) were added and the reaction mixture was transferred to an oxygen apparatus consisting of a plexiglass cylinder (27cm length x 10cm diameter). The apparatus was primed with oxygen (technical grade) for about 1 min and pressurized to 4 bar. The whole apparatus was shaken at room temperature, 170 rpm, 20h (vertical position of the MG5 vial). As for catalase, 20 µL of the stock solution [Catalase from *Micrococcus lysodeikticus* (ca. 170000 U/mL, Sigma Aldrich #60634), Catalase from Corynebacterium glutamicum (> 500000 U/mL, Sigma Aldrich #02071)] were used instead of horseradish peroxidase and 2,2'-azino-bis(3-ethylbenzthiazoline-6-sulfonic acid) (ABTS).

Various carbohydrate oxidases, more specifically galactose oxidase from *Fusarium* NRRL 2903 (M1 variant) (Escalettes et al., 2008), pyranose oxidase from *Phanerochaete chrysosporium* and hexose oxidase from *Chondrus crispus,* used in recombinant form in *E*. *coli* BL21(DE3) as lyophilized whole cells and obtained as described in Example 7, could be shown to convert the hydroxy groups of a range of cyclic substrates, including HMF, benzyl alcohol and *ortho*hydroxybenzyl, to the corresponding carbonyl groups.

### Example 2: Dependency of the oxidation step on pH and buffer concentration

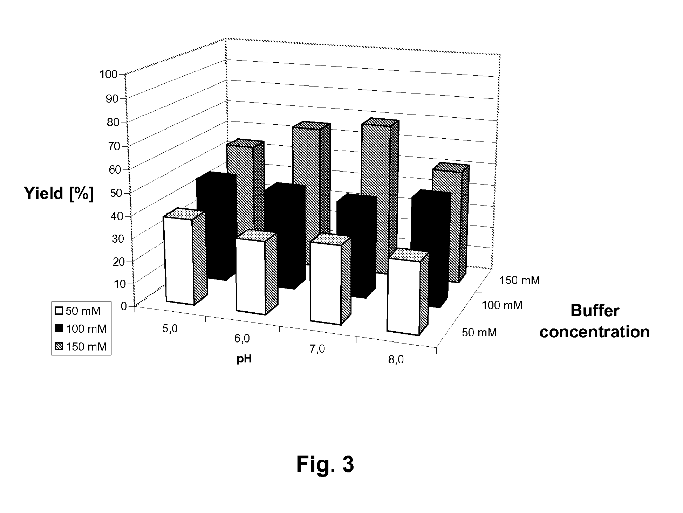

The reaction was carried out in aqueous solution in phosphate buffer using the M1 variant of galactose oxidase from *Fusarium* NRRL2903 under standard conditions, *i. e*. 40 mmol HMF, 10 mg whole-cell preparation of galactose oxidase, ABTS (30 µL, 10 mg/mL), horse radish peroxidase (30 µL, 10 mg/mL) and 100 mM sodium phosphate buffer pH 7. The oxygen pressure was 4 bar. The optimum pH was 7.0 for galactose oxidase (Tab. 2, Fig. 3) As for catalase, 20 µL of the stock solution [Catalase from *Micrococcus lysodeikticus* (ca. 170000 U/mL, Sigma Aldrich #60634), Catalase from Corynebacterium glutamicum (> 500000 U/mL, Sigma Aldrich #02071)] were used instead of horseradish peroxidase and 2,2'-azino-bis(3-ethylbenzthiazoline-6-sulfonic acid) (ABTS).

A similar suite of experiments was carried out and showed that the optimal pH value is 8.0 if pyranose oxidase is used. However, oxidation takes place over a wide pH range from 5.0 to 9.0 in the case of both enzymes.

### Example 3: Effect of various buffer concentrations on the oxidation steps

A range of experiments was carried out under standard conditions as specified in Example 1, except for the fact that various concentrations of buffer were used. It could be shown that higher buffer strengths led to an increase in conversion (**Fig. 3**).

### Example 4: Effect of elevated levels of oxygen on the oxidation step

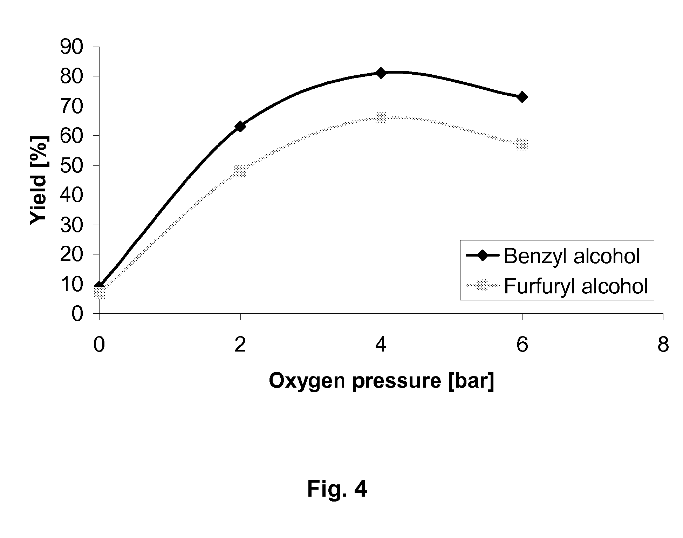

Pilot experiments were carried out to investigate the dependency of the reaction on the oxygen pressure applied. More specifically, 40 mmol of two substrates, benzyl alcohol and furfuryl alcohol, were subjected to oxidation by the galactose oxidase as specified in Example 1, horse radish peroxidase (30 µL, 10 mg/mL) and ABTS (30 µL, 10 mg/mL) in 100 mM sodium phosphate buffer pH 7. The application of oxygen at elevated pressure was shown to be optimal at 4 bar (**Fig. 4**).

### Example 5: One-pot transamination of oxidation products of various cyclic compounds comprising a hydroxyl group in the oxidation reaction mixture

Following incubation of the reaction mixture in Example 1 for 20 h, the transamination reaction mix consisting of transaminase, L-alanine, alanine dehydrogenase, NADH and the corresponding recycling system (see above) was added and the reaction was shaken for another 20 h. DFF was transaminated employing the recombinant transaminase from *Vibrio fluvialis* (*Vf*-ωTA) or *Paracoccus denitrificans* (pCR6) transaminases, see WO 2010/089171 for details of transaminases

**Tab. 2 Optimisation of pH und buffer concentration using the M1 variant of galactose oxidase from Fusarium NRRL2903.**

| **Entry** | **pH** | **buffer strength [mM]** | **yield [%]** |
|---|---|---|---|
| 1 | 5.0 | 50 | 38 |
| 2 | 6.0 | 50 | 32 |
| 3 | 7.0 | 50 | 34 |
| 4 | 8.0 | 50 | 31 |
| 5 | 5.0 | 100 | 46 |
| 6 | 6.0 | 100 | 44 |
| 7 | 7.0 | 100 | 42 |
| 8 | 8.0 | 100 | 47 |
| 9 | 5.0 | 150 | 54 |
| 10 | 6.0 | 150 | 65 |
| 11 | 7.0 | 150 | 69 |
| 12 | 8.0 | 150 | 51 |

The transaminases were used as lyophilized whole cell preparation (recombinantly in *E*. *coli*), alanine dehydrogenase from *Bacillus subtilis* was purified prior to use and formate dehydrogenase was obtained from Codexis (# 24.11, H62411.01), see Example 7.

Reaction conditions were as follows:
Whole cell preparation of galactose oxidase from *Fusarium* NRRL 2903 (20 mg, lyophilised dry weight) was resuspended in sodium phosphate buffer (500 µL, 100 mM, pH = 7.0, 3 mg/mL CuSO₄.5H₂O) at 30°C and 120 rpm, 30 min. Horseradish peroxidase (15 µL, 10 mg/mL), ABTS (15 µL, 10 mg/mL) and HMF (5 mg) were added and the reaction mixture was placed into the oxygen apparatus (vertical position, room temperature, 4 bar O₂, 170 rpm) for 20 h. The reaction mixture was transferred to Eppendorf vials. A solution of whole cell preparation of ω-transaminase from *Vibrio fluvialis* (20 mg, lyophilised dry weight) in P₁-buffer (500µL, 100 mM, pH = 7.0, 2 mM PLP, 200 mmol L-alanine, 140 mmol ammonium formate) was added, followed by formate dehydrogenase (20 µL, Codexis) and purified alanine dehydrogenase from *Bacillus subtilis* (20µL, 7.5mg protein /mL). The reaction mixture was shaken at 30°C and 120 rpm in a horizontal position for 20h. The apparatus was primed with oxygen for about 1 min and pressurized to 4 bar.The reaction could be reproduced using as carbohydrate oxidase pyranose and hexose oxidase. The reaction conditions were identical except for the pact that the pH value was adjusted to 8, and 1 mM FAD was added rather than CuSO₄.5H₂O.

This example illustrates that the transamination reaction is fully compatible with the conditions of the oxidation reaction mixture. Irrespective of the transaminase used, HMF was converted to DAMF at a yield of more than 80% after 20 h reaction time, more specifically 81 % and 83 % if Vf-ωTA or pCR6 transaminases were used. Other compounds were oxidised and transaminated at a total combined yield as shown in Tab. 3. DFF formed during the oxidation step was quantitatively converted to DAMF. Remaining HMF did not inhibit the transamination, but was converted to the corresponding hydroxymethylamine. In an analogous fashion, HMF was converted by all transaminases to [5-(aminomethyl)furan-2-yl]methanol (AMFM). The rate of conversion and the identity of the products were determined using GC-MS.

**Tab. 3: A range of substrates, see Fig. 2, were subjected to the sequential oxidation and transamination steps as described in examples 1 and 5.**

| **Substrate** | **Rate of conversion [%]** |
|---|---|
| **1** | 48 |
| **2** | 56 |
| **3** | 51 |
| **4** | Could not be extracted |
| **5** | 13 |
| **6** | 33 |
| **7** | 33 |

### Example 6: Simultaneous oxidation by carbohydrate oxidases and transamination of various cyclic compounds comprising a hydroxyl function

The same experimental setup as outlined in Example 1 was added, except for the fact that HMF was used as a substrate *and all reagents required for the oxidation and transamination were added right at the beginning, including* pyranose oxidase from *Phanerochaete chrysosporium, horseradish peroxidase and ABTS,* ω-transaminase from *Vibrio fluvialis, formate dehydrogenase and alanine dehydrogenase from Bacillus subtilis. 1 mM FAD was present and the pH was 8*. *The reaction was allowed to proceed for 24 h.*

The reaction was reproduced using as carbohydrate oxidase hexose oxidase under identical reaction conditions.

### Example 7: Analytical methods

### Sample preparation for HPLC-UV measurements without derivatisation.

The reaction mixture was heated to 95°C for 10 min to precipitate enzymes, diluted 1/1 with MeCN and centrifuged (13000 rpm, 3 min). Samples of 100 µL were taken, diluted with 1 mL H₂O/MeCN 1/1 containing 0.1% TFA and subjected to HPLC-UV analysis using the following setup. Column: Phenomenex LUNA 5µm C18(2) 100 A (250 x 4.60 mm); oven temp.: 30°C, flow: 0.25 mL/min, eluent A: H₂O (0.1% TFA), eluent B: MeCN (0.1% TFA); Gradient: 0-4 min 30% B, 4-30 min 30-100% B, 30-36 min 100% B, 36-46 min 30% B.

### Sample derivatisation for GC-MS measurements.

Samples of 500 µL were treated with DMAP (500 µL, 10% in MeOH). Ethyl chloroformate (200 µL) was added and the mixture was incubated at 60°C and 300 rpm for 2h. Extraction with dichloromethane (2x 500µL) and drying of the combined organic phase over Na₂SO₄ gave a sample which was subjected to GC-MS analysis under following conditions: Agilent 7890A GC system equipped with an Agilent 975C mass-selective detector (El 70 eV) and a HP-5-MS column (30m x 0.25 mm x 0.25 µm film) using helium as carrier gas at a flow of 0.55 mL/min. The following temperature program was used: 100 °C, hold for 0.5 min, 10 °C/min, to 300 °C.

### FMOC derivatisation for HPLC-UV measurements.

Sample preparation was performed to a standard procedure involving formation of FMOC derivatives in a glass HPLC vial, addition of borate buffer pH 9, addition of FMOC reagent and removing the FMOC reagent excess by way of addition of EVA reagent, addition of dilution buffer. The sample was subsequently subjected to HPLC analysis using the following conditions. Column: Phenomenex LUNA 5µm C18(2) 100 A (250 x 4.60 mm); oven Temp. 30°C, flow: 0.5 mL/min, eluent A: H₂O (0.1% TFA), eluent B: MeCN (0.1% TFA); Gradient: 0-2 min 30% B, 2-15 min 30-100% B, 15-25 min 100% B, 25-30 min 30% B.

### Example 8: Preparation of enzymes

Genes were optimized and synthesized by GeneArt and delivered in a pMAL vector with ampicillin resistence. Protein-encoding insert was cut via Ndel and Xhol restriction enzymes (Fermentas) and ligated in a pET21 a expression vector via T4 Ligase (Fermentas). If oxidases were used, enzymes were prepared using pET16b or pET21a plasmid constructs as follows: a fresh single colony from an LB agar plate supplemented with 100 mg/ml Ampicillin and used to grow *E*. *coli* strain BL21 comprising the respective plasmid was used to inoculate 50 ml of an over night culture supplemented with the same amount of Ampicillin. Following incubation of said culture over night at 120 rpm and 30 °C 4 ml of the over night culture was used to inoculate 600 ml of the same medium in a 3 I flask. The flask was shaken at 37 °C and 120 rpm until an OD550 of 0.6 was reached, i.e. approximately two to four hours. The expression of the respective enzyme was subsequently induced by addition of 0.6 ml IPTG (Isopropyl-b-D-thiogalactopyranosid, 238 mg/ml), followed by incubation over night at 20 °C and 120 rpm under vigorous shaking. Cells were then spun down at 8000 rpm and 4 °C for 20 minutes. The supernatant was disposed of, the pellet was washed using physiological NaCl solution, spun down and lyophilised.

If other enzymes were used, constructs comprising the pASK-IBA35+ (IBA Biotagnology, Germany) plasmid backbone were used. A fresh single colony from an LB agar plate supplemented with 100 mg/ml Ampicillin and used to grow *E. coli* strain BL21 comprising the respective plasmid was used to inoculate 50 ml of an over night culture supplemented with the same amount of Ampicillin. Following incubation of said culture over night at 180 rpm and 30 °C 4 ml of the over night culture was used to inoculate 2 I of the same medium in a 5 l flask. The flask was shaken at 37 °C and 120 rpm until an OD550 of 0.6 was reached, i.e. approximately two to four hours. The expression of the respective enzyme was subsequently induced by addition of 0.2 ml Anhydrotetracyclin (2 mg/ml), followed by incubation for three hours at 30 °C and 180 rpm under vigorous shaking. Cells were then spun down at 5000 rpm and 4 °C for 20 minutes. The supernatant was disposed of, the pellet was washed using physiological NaCl solution, spun down and lyophilised.

In the case of alanine dehydrogenase, 4 g of the lyophilised cells were resuspended in phosphate buffer (50 mM NaH₂PO₄, 300 mM NaCl, 10 mM Imidazol) and lysed using ultrasonication (1 s pulse followed by incubation for 4 s, repeated for a total of 10 minutes). The cell lysate was spun down twice (16000rpm, 20 min, 4 °C), filtered using a syringe filter and applied to a His-trap column having a bed volume of 5 ml. Protein binding unspecifically was removed by applying phosphate buffer (50 mM NaH₂PO₄, 300 mM NaCl) comprising 20 mM imidazole. Protein was eluted using the same buffer, with a gradient from 50 mM to 80 mM imidazole being applied, followed by application of 60 ml of the same buffer comprising 150 mM imidazole Fractions comprising pure enzyme were pooled, dialyses against phosphate buffer to remove imidazole and concentrated via Vivaspin tubes and centrifugation at 4000 rpm.

### References

T. El Hajj, A. Masroua, J. C. Martin, and G. Decotes, Bull. Soc. Chim. Fr., 1987, 855 - 60

F. M. Ausubel (1995), Current Protocols in Molecular Biology. John Wiley & Sons, Inc.

F. Escalettes; N. J. Turner, ChemBioChem 2008, 9, 857-860

R. Huang; W. Qi; R. Su; Z. He, Chem. Commun. 2009, 46, 1115-1117.

D. Koszelewski; I. Lavandera; D. Clay; G. M. Guebitz; D. Rozzell; W. Kroutil, Angew. Chem. Int. Ed. 2008, 47, 9337-9340.

P. D. Hanke, 2009, WO 2009/023174 A2; Chem. Abstr. 150:235749.

F. Koopman; N. Wierckx; J. H. de Winde; H. J. Ruijssenaars, Bioresource Technol. 2010, 101, 6291-6296.

M. P. J. van Deurzen; F. van Rantwijk; R. A. Sheldon, J. Carbohydr. Chem. 1997, 16, 299-309.

T. H. de Koker; M. D. Mozuch; D. Cullen; J. Gaskell; P. J. Kersten, Appl. Environ. Microbiol. 2004, 70, 5794-5800.

## Claims

1. A method comprising the steps
(a) contacting a hydrocarbon comprising a hydroxyl group with
a biological agent having oxygen-dependent and cofactor-dependent carbohydrate oxidase activity
in the presence of oxygen and carbohydrate oxidase cofactor, and
(b) contacting the hydrocarbon produced in step a) with
a biological agent having transaminase activity and
a biological agent having cofactor-dependent amino acid dehydrogenase activity
in the presence of amino acid dehydrogenase cofactor and the substrate amino acid of the amino acid dehydrogenase.

2. The method according to claim 1, wherein the hydrocarbon comprising a hydroxyl group comprises a 5 or 6 membered ring carrying at least one substituent selected from the group comprising -(CH₂)ₓ-OH, wherein x is 0 to 4.

3. The method according to any of claims 1 to 2, wherein the hydrocarbon comprising a hydroxyl group is selected from the group of compounds represented by formulae (I) or (II) 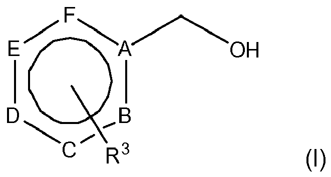 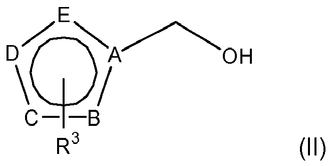 wherein up to two out of A, B, C, D, E and F are atoms each and independently selected from the group comprising N, S and O and the others are C,
wherein R³ is selected from the group comprising H, substituted or unsubstituted alkyl, alkenyl, alkynyl, -(CH₂)ₓ-CR⁴O, -(CH₂)ₓ-NH₂, -(CH₂)ₓ-NO₂, -(CH₂)ₓC-O-R⁴, -(CH₂)ₓ-CH₂SH, -(CH₂)ₓ-COOR⁴,
wherein x is 0 to 20, preferably 0 to 4,
and wherein R⁴ is selected from the group comprising H and substituted or unsubstituted alkyl, cycloalkyl, aryl and heteroaryl.

4. The method according to claim 3,
wherein up to two out of A, B, C, D, E and F are atoms each and independently selected from the group comprising N and O and the others are C,
wherein R³ is selected from the group comprising -(CH₂)ₓ-CR⁴O, -(CH₂)ₓ-NH₂, -(CH₂)ₓ-O-R⁴ and -(CH₂)ₓ-C-COOR⁴,
wherein x is 0 to 4,
and wherein R⁴ is selected from the group comprising H and alkyl comprising 1 to 4 carbon atoms.

5. The method according to claim 2, wherein the hydrocarbon comprising a hydroxyl group is a cycloalkanol, preferably cyclohexanol.

6. The method according to any of claims 1 to 5, wherein the oxygen-dependent and cofactor-dependent carbohydrate oxidase activity is an activity provided by an enzyme selected from the group comprising the M1 variant of galactose oxidase from *Fusarium* NRRL 2903, pyranose oxidase from *Phanerochaete chrysosporium,* hexose oxidase from *Chondrus crispus* and homologues thereof.

7. The method according to any of claims 1 to 6, wherein the amino acid dehydrogenase is alanine dehydrogenase.

8. The method according to any of claims 1 to 7, wherein the transaminase is selected from the group comprising the ω-transaminases from *Vibrio fluvialis* and *Paracoccus denitrificans* and homologues thereof.

9. The method according to any of claims 1 to 8, wherein step b) is carried out in the presence of a biological agent having formate dehydrogenase activity.

10. The method according to any of claims 1 to 9, wherein the redox factor produced by the biological agent having amino acid dehydrogenase activity is consumed by the biological agent having formate dehydrogenase activity.

11. The method according to any of claims 1 to 10, wherein step a) is carried out in the presence of an H₂O₂-degrading activity.

12. The method according to any of claims 1 to 11, wherein the H₂O₂-degrading activity is provided by a biological agent selected from the group comprising catalase and horse radish peroxidase/ABTS and homologues thereof.

13. The method according to any of claims 1 to 12, wherein steps a) and b) are carried out simultaneously in the same reaction mixture.

14. The method according to claim 13, wherein at least one component essential for the transaminase activity, preferably the biological agent having transaminase activity, is added to the reaction mixture following addition of the components essential for oxygen-dependent and cofactor-dependent carbohydrate oxidase activity.

15. The method according to any of claims 1 to 14, wherein the level of oxygen pressure is 2 to 7 bar, preferably 3 to 5 bar.

16. The method according to any of claims 1 to 15, wherein one or more, preferably all, of the biological agents selected from the group comprising the biological agent having oxygen-dependent and cofactor-dependent carbohydrate oxidase activity, the biological agent having transaminase activity and the biological agent having cofactor-dependent alanine dehydrogenase activity are associated with a viable cell.

17. A use of a mixture comprising a biological agent having oxygen-dependent and cofactor-dependent carbohydrate oxidase activity, a biological agent having transaminase activity, a biological agent having amino acid dehydrogenase activity, oxygen, the substrate amino acid of the amino acid dehydrogenase, carbohydrate oxidase cofactor and amino acid dehydrogenase cofactor and preferably a biological agent having formate dehydrogenase activity for transaminating a hydrocarbon comprising a hydroxyl group.

18. An aqueous reaction mixture comprising a hydrocarbon comprising a hydroxyl group, a biological agent having oxygen-dependent and cofactor-dependent carbohydrate oxidase activity, oxygen, carbohydrate oxidase cofactor, a biological agent having transaminase activity, a biological agent having amino acid dehydrogenase, amino acid dehydrogenase cofactor, the substrate amino acid of the amino acid dehydrogenase and preferably a biological agent having formate dehydrogenase activity, wherein all biological agents are or comprise a heterologous polypeptide having the respective activity of interest.

## Amended claims

### Amended claims in accordance with Rule 137(2) EPC.

**1.** A method comprising the steps
(a) contacting a hydrocarbon comprising a hydroxyl group with
a polypeptide having oxygen-dependent and cofactor-dependent carbohydrate oxidase activity or a whole-cell biocatalyst comprising such as polypeptide
in the presence of oxygen and carbohydrate oxidase cofactor, and (b) contacting the hydrocarbon produced in step a) with
a polypeptide having transaminase activity or a whole-cell biocatalyst comprising such a polypeptide and
a polypeptide having cofactor-dependent amino acid dehydrogenase activity or a whole-cell biocatalyst comprising such a polypeptide
in the presence of amino acid dehydrogenase cofactor and the substrate amino acid of the amino acid dehydrogenase,
wherein the hydrocarbon comprising a hydroxyl group comprises a 5 or 6 membered ring carrying at least one substituent selected from the group comprising -(CH₂)ₓ-OH, wherein x is 0 to 4, and
wherein the polypeptide having oxygen-dependent and cofactor-dependent carbohydrate oxidase activity is selected from the group comprising the M1 variant of galactose oxidase from *Fusarium* NRRL 2903, pyranose oxidase from *Phanerochaete chrysosporium,* hexose oxidase from *Chondrus crispus* and homologues thereof.

**2.** The method according to claim 1, wherein the hydrocarbon comprising a hydroxyl group
is selected from the group of compounds represented by formulae (I) or (II) 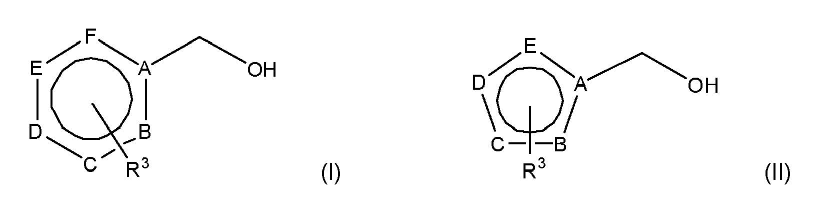 wherein up to two out of A, B, C, D, E and F are atoms each and independently selected from the group comprising N, S and O and the others are C,
wherein R³ is selected from the group comprising H, substituted or unsubstituted alkyl, alkenyl, alkynyl, -(CH₂)ₓ-CR⁴O, -(CH₂)ₓ NH₂, -(CH₂)ₓ NO₂, -(CH₂)ₓ O-R⁴, -(CH₂)x-CH₂SH, -(CH₂)ₓ-COOR⁴,
wherein x is 0 to 20, preferably 0 to 4,
and wherein R⁴ is selected from the group comprising H and substituted or unsubstituted alkyl, cycloalkyl, aryl and heteroaryl.

**3.** The method according to claim 2,
wherein up to two out of A, B, C, D, E and F are atoms each and independently selected from the group comprising N and O and the others are C,
wherein R³ is selected from the group comprising -(CH₂)ₓ-CR⁴O, -(CH₂)ₓ-NH₂, -(CH₂)ₓ-O-R⁴ and -(CH₂)ₓ COOR⁴,
wherein x is 0 to 4,
and wherein R⁴ is selected from the group comprising H and alkyl comprising 1 to 4 carbon atoms.

**4.** The method according to claim 3, wherein the hydrocarbon comprising a hydroxyl group is a cycloalkanol, preferably cyclohexanol.

**5.** The method according to any of claims 1 to 4, wherein the amino acid dehydrogenase is alanine dehydrogenase.

**6.** The method according to any of claims 1 to 5, wherein the transaminase is selected from the group comprising the ω-transaminases from *Vibrio fluvialis* and *Paracoccus denitrificans* and homologues thereof.

**7.** The method according to any of claims 1 to 6, wherein step b) is carried out in the presence of a polypeptide having formate dehydrogenase activity or a whole-cell biocatalyst comprising such as polypeptide.

**8.** The method according to any of claims 1 to 7, wherein the redox factor produced by the polypeptide having amino acid dehydrogenase activity or whole-cell biocatalyst comprising such as polypeptide is consumed by the polypeptide having formate dehydrogenase activity or whole-cell biocatalyst comprising such as polypeptide.

**9.** The method according to any of claims 1 to 8, wherein step a) is carried out in the presence of an H₂O₂-degrading activity.

**10.** The method according to any of claims 1 to 9, wherein the H₂O₂-degrading activity is provided by a polypeptide selected from the group comprising catalase and horse radish peroxidase/ABTS and homologues thereof or a whole-cell biocatalyst comprising such as polypeptide.

**11.** The method according to any of claims 1 to 10, wherein steps a) and b) are carried out simultaneously in the same reaction mixture.

**12.** The method according to claim 11, wherein at least one component essential for the transaminase activity, preferably the polypeptide having transaminase activity or thewhole-cell biocatalyst comprising such as polypeptide, is added to the reaction mixture following addition of the components essential for oxygen-dependent and cofactor-dependent carbohydrate oxidase activity.

**13.** The method according to any of claims 1 to 12, wherein the level of oxygen pressure is 2 to 7 bar, preferably 3 to 5 bar.

**14.** The method according to any of claims 1 to 13, wherein one or more, preferably all, of the polypeptides selected from the group comprising the polypeptide having oxygen-dependent and cofactor-dependent carbohydrate oxidase activity, the polypeptide having transaminase activity and the polypeptide having cofactor-dependent alanine dehydrogenase activity are associated with a viable cell.

**15.** A use of a mixture comprising a polypeptide having oxygen-dependent and cofactor-dependent carbohydrate oxidase activity or a whole-cell biocatalyst comprising such a polypeptide, a polypeptide having transaminase activity or a whole-cell biocatalyst comprising such a polypeptide, a polypeptide having amino acid dehydrogenase activity or a whole-cell biocatalyst comprising such a polypeptide, oxygen, the substrate amino acid of the amino acid dehydrogenase, carbohydrate oxidase cofactor and amino acid dehydrogenase cofactor and preferably a polypeptide having formate dehydrogenase activity or a whole-cell biocatalyst comprising such a polypeptide for transaminating a hydrocarbon comprising a hydroxyl group,
wherein the hydrocarbon comprising a hydroxyl group comprises a 5 or 6 membered ring carrying at least one substituent selected from the group comprising -(CH₂)ₓ-OH, wherein x is 0 to 4,
and wherein the polypeptide having oxygen-dependent and cofactor-dependent carbohydrate oxidase activity is selected from the group comprising the M1 variant of galactose oxidase from *Fusarium* NRRL 2903, pyranose oxidase from *Phanerochaete chrysosporium,* hexose oxidase from *Chondrus crispus* and homologues thereof.

**16.** An aqueous reaction mixture comprising a hydrocarbon comprising a hydroxyl group, a polypeptide having oxygen-dependent and cofactor-dependent carbohydrate oxidase activity or a whole-cell biocatalyst comprising such a polypeptide, oxygen, carbohydrate oxidase cofactor, a polypeptide having transaminase activity or a whole-cell biocatalyst comprising such a polypeptide, a polypeptide having amino acid dehydrogenase, amino acid dehydrogenase cofactor, the substrate amino acid of the amino acid dehydrogenase or a whole-cell biocatalyst comprising such as polypeptide and preferably a polypeptide having formate dehydrogenase activity or a whole-cell biocatalyst comprising such a polypeptide, wherein one ore more than one, preferably all said polypeptides or whole-cell biocatalysts are or comprise, respectively, a heterologous polypeptide having the respective activity of interest,
wherein the hydrocarbon comprising a hydroxyl group comprises a 5 or 6 membered ring carrying at least one substituent selected from the group comprising -(CH₂)ₓ-OH, wherein x is 0 to 4,
and wherein the polypeptide having oxygen-dependent and cofactor-dependent carbohydrate oxidase activity is selected from the group comprising the M1 variant of galactose oxidase from *Fusarium* NRRL 2903, pyranose oxidase from *Phanerochaete chrysosporium,* hexose oxidase from *Chondrus crispus* and homologues thereof.
